# FASCICULE DE BREVET EUROPEEN

(11) **EP 2 294 041 B1**
(45) Date de publication et mention de la délivrance du brevet: **25.07.2018**
(21) Numéro de dépôt: 09750159.7
(22) Date de dépôt: 18.05.2009
(51) Int. Cl.: C07C 29/92, C07C 31/22

(54) **PROCEDE DE PURIFICATION DU GLYCEROL BRUT**
VERFAHREN ZUR AUFREINIGUNG VON ROHEM GLYCERIN
METHOD OF PURIFYING CRUDE GLYCEROL

(30) Priorité: 19.05.2008 FR 0802689
(43) Date de publication de la demande: 16.03.2011
(73) Titulaire: Rhodia Poliamida E Especialidades Ltda, Sao Paulo - SP (BR)
(72) Inventeur: MACRET, Richard, 01232-010 Sao Paulo SP (BR); LOURENCO, Wagner Celio Ferraz, 13083-130 Campinas - Sao Paulo (BR)
(74) Mandataire: Ridray, Annabelle
(86) Numéro de dépôt international: PCT/IB2009/005635
(87) Numéro de publication internationale: WO 2009/141702

(56) Documents cités:
- WO-A-97/01523
- FR-A- 2 906 807
- GB-A- 933 714

## Description

La présente invention concerne un procédé de purification du glycérol brut obtenu à partir de matières premières telles que le glycérol obtenu au cours de la fabrication du biodiésel ou du glycérol obtenu au cours de transformations des graisses ou d'huiles. L'invention vise notamment à faire réagir le produit brut comprenant du glycérol avec des composés organiques spécifiques de type acétone.

### ART ANTERIEUR

Le glycérol, le 1,2,3-propanetriol, est présent sous forme combinée dans des huiles et graisses végétales et animales. Il est notamment présent sous forme de triglycérides combinés à des acides gras tels que les acides stéarique, oléique, palmitique et laurique. Le procédé industriel le plus répandu pour obtenir du glycérol à partir des huiles et graisses végétales et animales implique des réactions de saponification, d'hydrolyse haute pression et de transestérification avec des alcools, tels que l'éthanol ou le méthanol.

Le glycérol est également un sous-produit du biodiesel qui est obtenu généralement par la transestérification de glycérides par des alcools à chaînes courtes, par exemple le méthanol ou l'éthanol.

La réaction de transestérification est catalysée par un acide ou une base, selon les caractéristiques des huiles et/ou des graisses utilisées. Après la réaction de transestérification, les esters résultants sont séparés des réactifs en excès, du catalyseur et des sous-produits par un procédé comportant deux étapes. D'abord, on sépare le glycérol par décantation ou centrifugation, puis, on élimine les savons, les résidus de catalyseur et d'alcool par lavage à l'eau et barbotage ou utilisation de silicate de magnésium avec filtration. La production importante de biodiesel comme alternative aux sources fossiles s'accompagne d'une production élevée de glycérol obtenu comme sous-produit.

Selon les procédés de fabrication, le glycérol brut obtenu comprend des impuretés qui impliquent de nombreuses et complexes étapes de traitements.

A cette fin, il est notamment connu de purifier le glycérol brut par distillation en opérant avec des conditions particulières pour ne pas altérer le glycérol qui se décompose à des températures de 170-180 °C et qui peut se polymériser et générer des impuretés. Un tel procédé de purification n'est donc pas intéressant au niveau industriel.

Il existe ainsi un certain nombre de techniques complexes qui ont été développées dans le passé pour purifier du glycérol tout en évitant des décompositions ou autres réactions non désirées.

Par exemple, le brevet US 4,655,879 décrit un procédé de purification de glycérol brut très laborieux qui implique un grand nombre d'étapes dans lesquelles le glycérol brut est d'abord alcalinisé en présence d'air pour oxydation, puis distillé à de hautes températures sous pression réduites. Comme le glycérol obtenu présente une couleur non désirée, il est par ailleurs nécessaire de procéder à un traitement supplémentaire au charbon activé.

Le brevet US 4,990,695 décrit la purification du glycérol brut avec une combinaison d'opérations telles que l'ajustement du pH dans une gamme de 9 à 12, chauffage du milieu à 100°C, microfiltration et ensuite ultrafiltration. Le glycérol obtenu est alors distillé, éventuellement après un traitement avec des composés échangeurs d'ions.

FR 2 906 807 divulgue un procédé de synthèse d'acétals cycliques par réaction d'au moins un composé à fonction carbonyle choisi parmi les aldéhydes, les cétones sur un polyol, notamment le glycérol. L'acétal cyclique produit au cours de la réaction présente une solubilité dans l'eau inférieure à 20 000 mg/kg. Ainsi, simultanément à la réaction de synthèse dudit acétal cyclique, une partie de la phase organique contenant l'acétal cyclique est séparée de la phase continue aqueuse par extraction à l'aide d'un solvant organique au sein du réacteur Lorsqu'une méthode est utilisée un tiers corps « extractif » est ajouté ce qui rend le procédé plus onéreux. En outre, une étape supplémentaire d'évaporation sous vide est nécessaire pour récupérer l'acétal, ce qui est plus complexe.

Il est ainsi recherché le développement d'un procédé simple et industriel de purification du glycérol à partir de glycérol brut qui soit peu coûteux et dans des conditions usuelles de températures et de pression ; permettant d'obtenir du glycérol purifié ayant une qualité adéquate pour un certain nombre d'applications, tout en évitant les inconvénients mentionnés précédemment.

### INVENTION

Il a maintenant été mis en évidence qu'il était possible de purifier du glycérol brut par un procédé simple à mettre en oeuvre, efficace et qui par ailleurs n'altérait pas le glycérol ou sa couleur. Ce procédé consiste à générer un dioxolane par réaction du glycérol brut et l'acétone, de purifier ledit dioxolane par distillation pour ensuite le reconvertir en glycérol et en acétone.

Un tel procédé présente de nombreux avantages. En effet, ce procédé permet une excellente purification et séparation du glycérol quel que soit le type de glycérol brut utilisé, en termes d'impuretés et de pH. Par ailleurs, le dioxolane intermédiaire formé présente un point d'ébullition plus faible que le glycérol permettant de réaliser une distillation dans des conditions usuelles, industrielles et économiques, sans entrainer de dégradation du glycérol. Un autre avantage réside dans le fait que le dioxolane ne comprend pas de groupement hydroxyles libres contrairement au glycérol ce qui permet d'éviter les réactions secondaires et de polymérisation du glycérol, notamment lors de la distillation.

La présente invention concerne un procédé de purification de glycérol brut provenant de matières premières renouvelables consistant en les étapes suivantes successivement et les unes à la suite des autres :
(a) formation d'un dioxolane par réaction entre le glycérol brut et l'acétone;
(b) séparation du dioxolane formé du milieu réactionnel par distillation ;
(c) réversion du dioxolane pour former du glycérol et de l'acétone ;
(d) récupération du glycérol,
caractérisé en ce que l'étape b) consiste à procéder, sur une même colonne de distillation, à la distillation de l'acétone, puis une augmentation de la température afin de distiller l'eau, puis encore une augmentation de la température pour distiller le dioxolane formé. Le procédé de l'invention peut être réalisé en continu ou en discontinu. Les étapes mentionnées sont réalisées successivement et les unes à la suite des autres. Chacune des étapes du procédé peut être réalisée en continu ou en discontinu.

Le glycérol brut est obtenu à partir de matières premières renouvelables, en particulier le glycérol brut est obtenu au cours de la fabrication du biodiesel ou obtenu au cours de transformation de graisses ou d'huiles, particulièrement des graisses ou huiles animales ou végétales. Le glycérol brut est généralement obtenu par réaction de saponification, transestérification et/ou hydrolyse des graisses ou huiles animales ou végétales.

Le glycérol brut comprend généralement de 5 à 95 % en poids de glycérol, notamment de 40 à 90 % en poids de glycérol. Le glycérol brut comprend également des sels inorganiques, des glycérides, de l'eau et d'autres composés organiques.

Le glycérol brut peut éventuellement être traité pour le procédé de l'invention, notamment par exemple par ajustement du pH, filtration ou distillation.

Il est ainsi possible de filtrer le glycérol brut afin d'éliminer les matières organiques insolubles et/ou de le distiller généralement à des températures comprises entre 100 et 120°C à la pression atmosphérique pour éliminer de l'eau et les composés volatiles.

L'étape a) du procédé selon l'invention vise à former un dioxolane par réaction du glycérol contenu dans le glycérol brut et d'une cétone.

Le dioxolane selon l'invention est un cétal. Les acétals sont obtenus par addition nucléophile d'un alcool sur un aldéhyde en milieu acide, suivie d'une élimination d'eau. Les cétals sont obtenus par le même type de réaction effectuée sur les cétones.

La cétone utilisée dans la présente invention est l'acétone.

Selon le procédé utilisé, il est possible d'utiliser diverses proportions de glycérol et de cétone dans le milieu réactionnel. Par exemple en discontinu on peut utiliser un rapport molaire de 1 à 5 de cétone par rapport au glycérol. Dans un procédé continu, on peut par exemple utiliser du glycérol en boucle et ajouter de faibles proportions de cétone ; notamment de 5 à 20 % en mole.

Le cétal formé est un composé de type 1,3-dioxolane, répondant notamment à la formule générale (I) suivante : 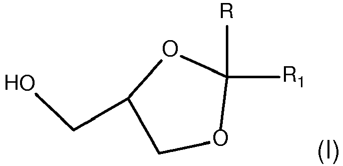 dans laquelle R et R₁ représentent un groupements méthyl.

On préfère notamment le 2,2-diméthyle-1,3-dioxolane-4-méthanol.

Le cétal formé présente préférentiellement une bonne solubilité dans l'eau, notamment supérieure à 20 000 mg/kg à température ambiante. Les cétones utilisés peuvent également présenter une solubilité supérieure à 20 000 mg/kg à température ambiante.

La réaction de formation du dioxolane est généralement conduite à une température comprise entre 50 et 150 °C, préférentiellement entre 60 et 80 °C.

Cette réaction peut être conduite en absence ou en présence de solvant.

Cette réaction peut être réalisée pendant 2 à 8 heures, généralement entre 3 et 6 heures.

Cette réaction est préférentiellement conduite en milieu acide, notamment avec un pH variant de 2,5 à 7,0, préférentiellement de 5,0 à 7,0, plus préférentiellement de 5,5 à 7,0.

On peut notamment utiliser des catalyseurs acides pour cette réaction, telles que des acides organiques ou inorganique ou leurs sels. On peut citer l'utilisation d'acide acétique, d'acide sulfurique, les résines échangeuses d'ion de type carboxylique ou sulfonique. Ces résines peuvent se présenter sur un lit fixe dans le réacteur.

En fin de réaction il est possible de neutraliser le catalyseur, notamment par ajout de carbonate de sodium ou hydroxyde de sodium.

La cétone n'ayant pas réagi peut être éliminée par simple distillation.

Une telle réaction entre le glycérol et une cétone ou un aldéhyde pour former un dioxolane est bien connue et est notamment mentionné dans les publications suivantes : R. J. Fessenden & J. F. Fessenden, Organic Chemistry, Second Edition, Page 524, 1982 and T. W. Greene, Protective Groups in Organic Chemistry, John Wiley & Sons, 1981.

L'étape b) vise à la séparation du dioxolane formé du milieu réactionnel par distillation, préférentiellement sous pression réduite.

Pour procéder à la distillation, on distille les différents composés sur une même colonne de distillation en faisant varier la température, et éventuellement la pression. On procède à la distillation de la cétone, puis une augmentation de la température afin de distiller l'eau, puis encore une augmentation de la température pour distiller le dioxolane formé.

On utilise habituellement des températures comprises entre 60 et 190°C, et des pressions comprises entre 2 et 1000 mbars.

Le dioxolane obtenu présente généralement une pureté comprise entre 97 et 99 %, et peut comprendre de petites quantités de sels, de glycérides et/ou d'esters d'acides gras.

L'étape c) vise à la réaction de réversion du dioxolane en glycérol et cétone, notamment par catalyse acide en présence d'eau.

Cette réaction peut être effectuée de manière continue ou discontinue. La réaction peut notamment être une catalyse homogène ou hétérogène.

Les catalyseurs utilisés pour cette réaction peuvent être des acides organiques ou inorganiques ou leurs sels. On peut notamment citer l'utilisation d'acide acétique, d'acide sulfurique, les résines échangeuses d'ion de type carboxylique ou sulfonique. Ces résines peuvent se présenter sur un lit fixe dans le réacteur.

On utilise généralement de 0,5 à 1,0 % en poids, préférentiellement de 0,5 à 0,7 % en poids de catalyseur par rapport au poids de la masse réactionnelle.

La température de la réaction de réversion peut être comprise entre 25 et 150°C. La cétone peut être récupérée par distillation, notamment sous pression réduite éventuellement sous azote.

Le glycérol purifié obtenu selon l'invention présente notamment une pureté comprise entre 95 et 99,5 %.

D'autres détails ou avantages de l'invention apparaîtront plus clairement au vu des exemples donnés ci-dessous uniquement à titre indicatif.

### PARTIE EXPERIMENTALE

### Exemple 1

1000 g de produit brut, obtenu par réaction de transestérification d'huile de soja, comprenant 85 % en poids de glycérol, 6 % en poids d'eau ainsi que des glycérides des sels et autres impuretés, et ayant un pH de 5,5-6,0 (dans une solution aqueuse à 10 % en poids) est filtré afin d'éliminer de faibles quantités de matières grasses en suspension.

2145 g d'acétone sont ajoutés et le milieu de réaction est chauffé à reflux à une température de 65°C pendant 4 heures, puis refroidis à température ambiante et neutralisé par addition d'une solution aqueuse de carbonate de sodium.

L'excès d'acétone est récupéré par distillation à une température de 60-70°C à la pression atmosphérique et recyclé sans traitement. L'eau formée durant la réaction est distillée à une température de 100-120°C à la pression atmosphérique. Le 2,2-diméthyle-1,3-dioxolane-4-méthanol est récupéré par distillation à une température de 190°C à la pression atmosphérique.

Une analyse par chromatographie en phase vapeur révèle une pureté en 2,2-diméthyle-1,3-dioxolane-4-méthanol supérieure à 98 %.

### Exemple 2

Une expérimentation similaire à celle de l'exemple 1 est réalisée en utilisant cette fois 2 kg de produit brut comprenant 42 % en poids de glycérol, 14 % en poids d'eau et une grande quantité de produits volatiles (méthanol, éthanol). L'acétone est utilisé dans le même rapport molaire que dans l'exemple 1 et le 2,2-diméthyle-1,3-dioxolane-4-méthanol obtenu possède une pureté de 98 %.

### Exemple 3

3,5 kg de glycérol brut sous forme de pâte a une température de 25°C, obtenu par réaction de transestérification de graisses animales et de méthanol, comprenant 80 % en poids de glycérol, des glycérides et des sels inorganiques et ayant un pH de 11,7 (dans une solution aqueuse à 10 % en poids) est chauffé à une température de 60-70 °C pour obtenir un liquide visqueux. Une solution aqueuse à 50 % en poids d'acide sulfurique (422,3 g) est ajoutée au milieu pour obtenir un pH de 3,0. Les acides gras libres et autres impuretés sont séparés par filtration (541,2 g) et une solution liquide (3335,4 g) est obtenue.

Une réaction avec l'acétone est alors réalisée dans des conditions similaires à celles de l'exemple 1 et du 2,2-diméthyle-1,3-dioxolane-4-méthanol est obtenu avec une pureté de 97,5 %.

### Exemple 4 - comparatif

3,5 kg de produit brut similaire à celui de l'exemple 1 est chauffé à une température de 100°C sous une pression de 100 mm Hg afin de diminuer la teneur en eau et de composés volatiles.

12,9 kg de MIBK (méthyle isobutyl cétone) sont alors ajoutés et le milieu de réaction est chauffé à reflux à une température de 110°C pendant 5 heures avec un apport constant de MIBK et de suppression d'eau. Le milieu de réaction est alors refroidit a température ambiante et une partie de l'excès de MIBK est séparé par décantation. La masse réactionnelle est neutralisée avec une solution aqueuse d'hydroxyde de sodium.

L'excès de MIBK est supprimé par distillation à une température de 110-140°C et sous pression atmosphérique, suivit par une distillation du milieu à une température de 160°C et une pression de 200 mm Hg, permettant d'obtenir du 2-iso-butyl-2-méthyl-1,3-dioxolane-4-méthanol avec une pureté de 98 %.

### Exemple 5

Dans un réacteur de 27 litres, il est ajouté 16 kg de 2,2-diméthyl-1,3-dioxolane-4-méthanol, 3,2 kg d'eau et 4,8 g d'acide sulfurique à 98 %. Le milieu est mélangé à température ambiante pendant 1 heure. Une analyse de l'eau montre qu'après 1 heure, 50 à 55 % en poids du 2,2-diméthyl-1,3-dioxolane-4-méthanol est convertit en glycérol et acétone. L'acétone formée est enlevée du milieu par distillation à une température de 160°C et une pression de 100 mm Hg.

Le glycérol obtenu est limpide et présente une pureté de 98 %.

### Exemple 6 - comparatif

Dans un réacteur de 10 litres, il est ajouté 7 kg de 2-iso-butyl-2-méthyl-1,3-dioxolane-4-méthanol, 0,5 kg d'eau et 1,5 g d'acide sulfurique à 98 %. Le milieu est mélangé à température ambiante pendant 1,5 heure. Une analyse de l'eau montre qu'après 1 heure, 45 à 50 % en poids du 2-iso-butyl-2-méthyl-1,3-dioxolane-4-méthanol est convertit en glycérol et MIBK. La MIBK formée est enlevée du milieu par distillation à une température de 160°C et une pression de 200 mm Hg.

Après suppression complète du MIBK du milieu, l'eau en excès est distillée à une température de 100°C et une pression de 100 mm Hg.

Le glycérol obtenu est limpide et présente une pureté de 95 %.

## Revendications

1. Procédé de purification de glycérol brut provenant de matières premières renouvelables consistant en les étapes suivantes successivement et les unes à la suite des autres:
(a) formation d'un dioxolane par réaction entre le glycérol brut et l'acétone;
(b) séparation du dioxolane formé du milieu réactionnel par distillation ;
(c) réversion du dioxolane pour former du glycérol et l'acétone;
(d) récupération du glycérol
**caractérisé en ce que** l'étape b) consiste à procéder, sur une même colonne de distillation, à la distillation de l'acétone, puis une augmentation de la température afin de distiller l'eau, puis encore une augmentation de la température pour distiller le dioxolane formé.

2. Procédé selon la revendication 1, **caractérisé en ce que** le glycérol brut est obtenu au cours de la fabrication du biodiesel ou au cours de transformation de graisses ou d'huiles, particulièrement des graisses ou huiles animales ou végétales.

3. Procédé selon l'une quelconque des revendications précédentes, **caractérisé en ce que** la réaction de l'étape a) est une addition nucléophile en milieu acide.

4. Procédé selon l'une quelconque des revendications précédentes, **caractérisé en ce que** le milieu réactionnel de l'étape a) comprend un catalyseur acide.

5. Procédé selon l'une quelconque des revendications précédentes, **caractérisé en ce que** le milieu réactionnel de l'étape a) comprend un catalyseur acide choisi dans le groupe comprenant l'acide acétique, l'acide sulfurique, et les résines échangeuses d'ion de type carboxylique ou sulfonique.

6. Procédé selon l'une quelconque des revendications précédentes, **caractérisé en ce que** la distillation de l'étape b) est réalisée sous pression réduite.

7. Procédé selon l'une quelconque des revendications précédentes, **caractérisé en ce que** la distillation de l'étape b) est réalisée à une température comprise entre 60 et 190°C.

8. Procédé selon l'une quelconque des revendications précédentes, **caractérisé en ce que** la réaction de l'étape c) est une catalyse acide effectuée en présence d'eau.

9. Procédé selon l'une quelconque des revendications précédentes, **caractérisé en ce que** le milieu réactionnel de l'étape c) comprend un catalyseur acide.

10. Procédé selon l'une quelconque des revendications précédentes, **caractérisé en ce que** le milieu réactionnel de l'étape c) comprend un catalyseur acide choisi dans le groupe comprenant l'acide acétique, l'acide sulfurique, et les résines échangeuses d'ion de type carboxylique ou sulfonique.

## Patentansprüche

1. Verfahren zur Aufreinigung von rohem Glycerin aus nachwachsenden Rohstoffen, bestehend aus den folgenden Schritten, die in Folge und einer nach dem anderen durchgeführt werden:
(a) Bilden eines Dioxolans durch Reaktion zwischen rohem Glycerin und Aceton;
(b) Abtrennen des gebildeten Dioxolans aus dem Reaktionsmedium durch Destillation;
(c) Rückführen des Dioxolans zur Bildung von Glycerin und Aceton;
(d) Rückgewinnen des Glycerins
**dadurch gekennzeichnet, dass** Schritt b) darin besteht, die Destillation des Acetons auf derselben Destillationskolonne vorzunehmen, dann die Temperatur zu erhöhen, um das Wasser zu destillieren, und dann die Temperatur weiter zu erhöhen, um das gebildete Dioxolan zu destillieren.

2. Verfahren nach Anspruch 1, **dadurch gekennzeichnet, dass** das rohe Glycerin bei der Herstellung von Biodiesel oder bei der Verarbeitung von Fetten oder Ölen, insbesondere tierischen oder pflanzlichen Fetten oder Ölen, gewonnen wird.

3. Verfahren nach einem der vorangehenden Ansprüche, **dadurch gekennzeichnet, dass** es sich bei der Reaktion von Schritt a) um eine nukleophile Addition in einem sauren Medium handelt.

4. Verfahren nach einem der vorangehenden Ansprüche, **dadurch gekennzeichnet, dass** das Reaktionsmedium von Schritt a) einen sauren Katalysator umfasst.

5. Verfahren nach einem der vorangehenden Ansprüche, **dadurch gekennzeichnet, dass** das Reaktionsmedium von Schritt a) einen sauren Katalysator umfasst, ausgewählt aus der Gruppe, bestehend aus Essigsäure, Schwefelsäure und Ionenaustauschharzen von Carbon- oder Sulfonsäure.

6. Verfahren nach einem der vorangehenden Ansprüche, **dadurch gekennzeichnet, dass** die Destillation von Schritt b) unter vermindertem Druck durchgeführt wird.

7. Verfahren nach einem der vorangehenden Ansprüche, **dadurch gekennzeichnet, dass** die Destillation von Schritt b) bei einer Temperatur zwischen 60 und 190°C durchgeführt wird.

8. Verfahren nach einem der vorangehenden Ansprüche, **dadurch gekennzeichnet, dass** es sich bei der Reaktion von Schritt c) um eine in Gegenwart von Wasser durchgeführte Säurekatalyse handelt.

9. Verfahren nach einem der vorangehenden Ansprüche, **dadurch gekennzeichnet, dass** das Reaktionsmedium von Schritt c) einen sauren Katalysator umfasst.

10. Verfahren nach einem der vorangehenden Ansprüche, **dadurch gekennzeichnet, dass** das Reaktionsmedium von Schritt c) einen sauren Katalysator umfasst, ausgewählt aus der Gruppe, bestehend aus Essigsäure, Schwefelsäure und Ionenaustauschharzen von Carbon- oder Sulfonsäure.

## Claims

1. Process for the purification of crude glycerol originating from renewable starting materials consisting of the following stages, successively and one after the others:
(a) formation of a dioxolane by reaction between the crude glycerol and acetone;
(b) separation of the dioxolane formed from the reaction medium by distillation;
(c) reversion of the dioxolane in order to form glycerol and acetone;
(d) recovery of the glycerol;
**characterized in that** stage b) consists in carrying out, on one and the same distillation column, the distillation of the acetone, then an increase in the temperature in order to distil the water and then a further increase in the temperature in order to distil the dioxolane formed.

2. Process according to Claim 1, **characterized in that** the crude glycerol is obtained during the manufacture of biodiesel or during transformation of fats or oils, particularly animal or vegetable fats or oils.

3. Process according to either one of the preceding claims, **characterized in that** the reaction of stage a) is a nucleophilic addition in an acidic medium.

4. Process according to any one of the preceding claims, **characterized in that** the reaction medium of stage a) comprises an acid catalyst.

5. Process according to any one of the preceding claims, **characterized in that** the reaction medium of stage a) comprises an acid catalyst chosen from the group consisting of acetic acid, sulfuric acid and ionexchange resins of carboxylic or sulfonic type.

6. Process according to any one of the preceding claims, **characterized in that** the distillation of stage b) is carried out under reduced pressure.

7. Process according to any one of the preceding claims, **characterized in that** the distillation of stage b) is carried out at a temperature of between 60°C and 190°C.

8. Process according to any one of the preceding claims, **characterized in that** the reaction of stage c) is an acid catalysis carried out in the presence of water.

9. Process according to any one of the preceding claims, **characterized in that** the reaction medium of stage c) comprises an acid catalyst.

10. Process according to any one of the preceding claims, **characterized in that** the reaction medium of stage c) comprises an acid catalyst chosen from the group consisting of acetic acid, sulfuric acid and ionexchange resins of carboxylic or sulfonic type.
